# FASCICULE DE BREVET EUROPEEN

(11) **EP 2 950 938 B1**
(45) Date de publication et mention de la délivrance du brevet: **27.03.2019**
(21) Numéro de dépôt: 14708595.5
(22) Date de dépôt: 31.01.2014
(51) Int. Cl.: B05D 3/10, B05D 7/00, C08G 18/64, C08J 7/04

(54) **PROCEDE DE RECOUVREMENT DE SURFACES**
VERFAHREN ZUR BESCHICHTUNG VON OBERFLÄCHEN
METHOD FOR COATING SURFACES

(30) Priorité: 01.02.2013 FR 1350902
(43) Date de publication de la demande: 09.12.2015
(73) Titulaire: Natvi SAS, 91100 Corbeil-Essonnes (FR)
(72) Inventeur: MICHELOT, Robert, F-92160 Antony (FR)
(74) Mandataire: Monni, Richard
(86) Numéro de dépôt international: PCT/FR2014/050175
(87) Numéro de publication internationale: WO 2014/118473

(56) Documents cités:
- GB-A- 2 163 436
- TW-A- 200 925 700
- US-A- 5 037 677

## Description

La présente invention concerne le domaine technique des procédés de recouvrement de surfaces.

La fonctionnalisation de la surface des matériaux utilisés pour diverses applications, notamment dans le domaine médical, est un facteur déterminant pour l'interaction des matériaux avec l'environnement. Toutefois, une telle modification n'est pas toujours simple.

L'enrobage des matériaux à usage médical, qui sont généralement plus ou moins hydrophobes, est souvent difficile lorsqu'il faut déposer sur la surface de ceux-ci une solution aqueuse comprenant un produit biocompatible. En effet, le mouillage de la surface de ces matériaux, du fait de leur hydrophobicité, est imparfait et la solution aqueuse a tendance à adhérer de manière non uniforme, et à se rassembler en gouttelettes.

Afin de limiter ces phénomènes, différents traitements de surface sont généralement mis en oeuvre, afin de recouvrir les surfaces hydrophobes par des substances ou des composés hydrophiles, permettant d'assurer un bon mouillage de la surface, et de limiter le rassemblement sous forme de gouttelettes.

A ce jour plusieurs procédés de recouvrement de surfaces hydrophobes sont utilisés. Un premier type de procédé consiste en l'activation de la surface à recouvrir et le greffage covalent de molécules hydrophiles biocompatibles.

Un traitement préalable par un réactif susceptible de modifier la surface peut conduire à la formation de groupements dérivables. Par exemple, l'hydrolyse des polyesters ou des polyamides par une base forte ou un acide fort conduit à des groupements alcool, acides ou amines [Chollet, C. et al. 2007 Biomolecular Engeniering 24, 477-482]

Dans le cas d'un matériau inerte chimiquement, la surface peut être activée par des méthodes plus drastiques en utilisant, par exemple un plasma sous vide ou à pression ambiante ou par une radiation ionisante ou encore en solution, par action de réactifs générateurs de radicaux libres. Puis les ions ou les radicaux libres transitoires peuvent fixer, par liaisons covalentes ou ioniques des molécules biocompatibles [Goddard, J.M ; & Hotchkiss J.H. 2007 Progress in Polymer Science 32, 698-625].

Un second type de procédé, notamment illustré dans la demande US 2010/0076546, ou le brevet US5037677 consiste à recouvrir la surface hydrophobe par une solution de monomère d'une molécule hydrophile polymérisable. L'activation de la polymérisation est réalisée par irradiation UV, emploi de catalyseur ou une élévation de température.

De tels procédés sont toutefois difficiles à mettre en oeuvre et sont relativement onéreux. Par ailleurs, l'utilisation de catalyseurs, de plasma ou encore de rayons UV ou ionisants peuvent altérer la surface des matériaux, ce qui, dans le cadre des dispositifs à usage médical, peut conduire à la dégradation ou au dérèglement du dispositif.

Aussi, il existe un besoin de disposer d'une méthode simple, peu onéreuse, et n'altérant pas les propriétés de la surface à recouvrir par un composé hydrophile bio compatible.

Un troisième type de procédé a été est mis en oeuvre, et tire profit des capacités d'auto assemblage de certains composés en solution ou à l'interface liquide-solide ou liquide-gaz. Toutefois, dans la cadre de ce type de procédés, les molécules capables de s'auto assembler réagissent par leur partie polaire avec la surface à recouvrir, exposant ainsi leur partie hydrophobe. Lorsque l'extrémité polaire de ces molécules est liée au substrat, ce qui est généralement obtenu par liaison ionique ou covalente, les interactions entre ces molécules réorganisent l'édifice superficiel principalement par des forces de faible intensité. Ces interactions de faible énergie sont capables de former des fibrilles organisées en rangées à la surface du substrat. La solidité de cette monocouche provient donc à la fois d'interactions covalentes ou ioniques avec le matériau et d'interactions entre les molécules elles-mêmes.

Toutefois, même si un tel procédé est plus simple que ceux susmentionnés, il a pour conséquence d'augmenter l'hydrophobicité de la surface recouverte du fait de l'exposition des parties hydrophobes des molécules auto assemblées.

Aussi, les besoins susmentionnés demeurent.

Un objet de la présente invention est de pallier les inconvénients précités.

De plus, un objet de l'invention est de fournir un procédé simple permettant de recouvrir une surface hydrophobe par une substance hydrophile biocompatible.

Un des buts de l'invention est le traitement de surface des dispositifs médicaux afin de les rendre biocompatibles, lubrifiés et, éventuellement, capables de libérer des facteurs chimiques ou biologiques favorables à leur implantation (antibiotiques, molécules de croissance, facteurs de reconnaissance cellulaire etc.).

Un autre objet de l'invention vise à fournir des surfaces recouvertes, présentant un mouillage amélioré.

Encore un autre objet de l'invention a pour but de fournir des kits permettant de mettre en oeuvre le procédé susmentionné.

L'invention concerne un procédé de recouvrement d'une surface hydrophobe par une composition hydrophile, ledit procédé comprenant :
a. une étape de mise en contact de la surface hydrophobe en vue de son recouvrement avec une première composition comprenant
   - un solvant, et
   - un soluté consistant en un composé amphiphile capable de s'auto assembler et d'interagir avec ladite surface, l'auto-assemblage dudit composé et l'interaction avec la surface se faisant au moyen de liaisons différentes de liaisons covalentes ou ioniques,
      ledit solvant étant compatible avec ledit composé et ladite surface hydrophobe,
b. une étape subséquente de rinçage avec une solution aqueuse de ladite surface hydrophobe recouverte à l'étape précédente, et
c. une étape de mise en contact de la surface rincée avec une seconde composition hydrophile comprenant un polymère hydrophile.

L'invention est basée sur la constatation surprenante, faite par les inventeurs, que les composés amphiphiles auto assemblables sont capables, notamment en solution dans un solvant approprié, de former des couches de molécules orientées, capable d'interagir avec un support inerte sans pour autant former des liaisons covalentes ou ioniques avec le substrat. Ainsi, il n'est plus nécessaire d'activer le substrat, ce qui limite sa dégradation.

Le composé utilisé dans le cadre de l'invention est un composé amphiphile, c'est-à-dire un composé qui possède à la fois au moins un groupe hydrophile et au moins un groupe hydrophobe.

L'auto assemblage moléculaire est le phénomène par lequel des composés forment par eux-mêmes des structures avec un haut degré d'organisation, sans intervention externe. On peut schématiquement considérer que ces composés possèdent une affinité suffisante pour qu'ils s'assemblent entre eux.

On distingue deux types d'auto-assemblage, intramoléculaire et intermoléculaire. L'auto-assemblage intramoléculaire produit souvent des polymères complexes qui ont la possibilité d'adopter une conformation de structure stable et bien définie. L'autoassemblage intermoléculaire définit la capacité que certaines molécules ont de former des assemblages supramoléculaires. Un exemple de ce type d'auto assemblage concerne la formation de micelles à partir de surfactants en solution. L'auto assemblage est généralement dû à des liaisons de type van der Waals. La stabilité de l'auto assemblage résulte en grande partie de facteurs thermodynamiques qui privilégient les formes ordonnées de la matière (néguentropie) par rapport aux états désordonnés.

L'auto-assemblage peut se produire spontanément, par exemple dans les cellules (où la membrane est faite d'une double couche lipidique auto-assemblée) et d'autres systèmes biologiques, tout comme dans les systèmes artificiels. Il en résulte généralement une augmentation de l'organisation interne du système. Les systèmes biologiques auto-assemblés, incluant les peptides synthétiques auto-assemblés et d'autres biomatériaux, montrent une plus grande facilité de manipulation, de biocompatibilité et de fonctionnalité. Ces avantages sont dus directement à l'auto-assemblage à partir de précurseurs biocompatibles qui créent des biomatériaux synthétisés à l'échelle nanométrique.

Dans la première étape du procédé, seules les interactions intermoléculaires sont mises en jeu. Les forces causées par l'auto assemblage de ces composés sont suffisantes pour permettre au composé d'interagir avec le substrat, et de rester « fixé » à celui-ci, sans qu'il soit nécessaire de faire intervenir des interactions physico-chimiques avec celui-ci.

En d'autres termes, il n'existe pas de liaison covalente entre le composé auto assemblable et la surface recouverte par celui-ci. De la même manière, il n'existe pas de liaisons ioniques entre le composé auto assemblable et la surface recouverte par celui-ci.

Dans la première étape du procédé selon l'invention, une solution de solvant comprenant un composé auto assemblable est mise en contact avec la surface à recouvrir. « ledit solvant est compatible avec ledit composé et ladite surface hydrophobe » ce qui signifie que le composé auto assemblable est soluble dans ledit solvant, et que le solvant n'exerce pas d'effet altérant drastiquement la nature et la surface du substrat à recouvrir.

Dans un mode de réalisation avantageux, le solvant utilisé est susceptible d'exercer une légère abrasion de la surface à recouvrir, de sorte que des micro-dépressions peuvent être provoquées à la surface de ladite surface.

Avantageusement, la surface est préalablement nettoyée et dégraissée afin d'éliminer des traces de composés pouvant altérer l'auto assemblage des composés.

Selon la nature de la surface, elle peut être nettoyée et dégraissée avec un solvant approprié. L'homme de métier est capable de déterminer le solvant ou les mélanges de solvants requis. La partie exemple, ci-après donne certaines indications.

Dans l'invention, on entend par « solvant compatible avec ledit composé» un solvant capable de solubiliser ledit composé amphiphile auto assemblable. L'homme de métier peut aisément déterminer le solvant, ou les mélanges de solvants les plus appropriés pour solubiliser le composé amphiphile. On entend par « solvant compatible avec ladite surface hydrophobe» un solvant capable de ne pas dégrader ou altérer la surface hydrophobe.

Dans un mode de réalisation de l'invention, la première étape du procédé peut être mise en oeuvre directement lors de la fabrication de la surface hydrophobe à recouvrir. Par exemple, le composé auto assemblable, considéré comme un additif sous la forme de poudre ou de granules, éventuellement sous forme d'une solution, notamment d'une solution fortement concentrée, est ajouté à des granulés de polymères hydrophobes, le mélange ainsi obtenu est alors soumis à une fusion puis une extrusion. Dans ce mode de réalisation, lors du refroidissement, les composés auto assemblables migrent lentement vers les parties superficielles de la surface en formation, et les parties polaires du composé sont exposées à l'extérieur de la surface.

Dans encore un autre mode de réalisation avantageux, la surface à recouvrir est traitée avec un solvant du polymère, pendant un temps déterminé, de telle sorte qu'une micro abrasion apparait sur les parties superficielles de la surface. De telles abrasions permettent la diffusion et le regroupement du composé auto assemblable et l'exposition de sa partie hydrophile vers l'extérieur du substrat.

Dans la seconde phase, un composé ou polymère biocompatible sous forme de gel dilué ou de polymère très dispersé vient enrober le substrat déjà recouvert par des molécules auto assemblables.

Les surfaces hydrophobes à recouvrir par le procédé de l'invention sont généralement en matériaux polymères du type thermoplastique comme le polyéthylène (PE), le polypropylène (PP), le polyéthylènetéréphtalate (PET), le polybutylène théréphtalate (PBT), le polyoxyméthylène (POM), le polyarylate (PAr), polyéthercétone (PEK), les polymères fluorés (par exemple : polyfluorure de vinylidène (PVDF), les polymères cyclo-oléfiniques (COC) (par exemple les polymères commercialisés sous la marque « TOPAS »), les copolymères cyclooléfiniques (COP) (par exemple les copolymères commercialisés sous la marque «Zeonex»), polytétrafluoroéthylène (PTFE), le polychlorotrifluoroéthylène (PCTFE)), polysulfone (PSU), l'éthylène-propylène-diène monomère (EPDM), ou de type thermodurcissable comme les caoutchoucs synthétiques (par exemple: les caoutchouc halogénobutyles, les caoutchoucs nitriles, les polyisoprènes, et les polychloroprène) ou encore du type élastomères thermoplastiques (par exemple : le copolymère EPDM-PP commercialisé sous la marque « Santoprène », le copolymère bloc styrène-éthylène-butylène-styrène (SEBS), etc.).

D'autres surfaces telles que l'acier inoxydable, l'or, le titane, le platine, l'aluminium, l'alliage de nickel et de titane ou nitinol, le tantale, les silicones peuvent être recouvertes selon le procédé selon l'invention. La liste susmentionnée n'est pas limitative, et l'homme de métier comprend qu'il s'agit des surfaces hydrophobes utilisables notamment dans le cadre des dispositifs à usage médical.

Dans une seconde étape du procédé selon l'invention, la surface recouverte par le composé amphiphile susmentionné est lavée avec une solution aqueuse, notamment de l'eau, avantageusement distillée, en particulier stérile, ou un mélange de solvants avec l'eau.

Une telle étape de lavage est essentielle car elle améliore l'organisation des molécules auto assemblables à la surface du substrat.

Une fois lavée, la surface peut avantageusement être séchée.

Dans la troisième étape du procédé selon l'invention, la surface hydrophobe, recouverte par le composé amphiphile auto assemblable qui a été lavée, et éventuellement séchée est recouverte, ou enduite, par une seconde composition comprenant au moins un, ou plusieurs, polymère hydrophiles.

La seconde composition comprenant au moins un, ou plusieurs, polymères hydrophiles est classiquement mise en solution, et mise en contact avec la surface préalablement lavée et éventuellement séchée à l'étape précédente.

Les secondes compositions comprenant au moins un polymère hydrophile avantageuses selon l'invention comprennent, sans que cela soit limitatif, des polymères anioniques ou cationiques, des polymères acides, des polymères d'amines ou d'acides aminés, et des sels biologiquement et/ou pharmaceutiquement acceptables de ceux-ci. Il est possible également de disposer de mélanges des polymères susmentionnés. On peut citer comme exemples : le collagène, le collagène modifié par oxydation, les polysaccharides, les alginates, l'acide hyaluronique, la polylysine ... De tels exemples sont à titre indicatif et ne doivent pas être considérés comme limitant la portée de l'invention.

Il est avantageux dans l'invention de choisir le polymère hydrophile de sorte qu'il présente une affinité pour le composé auto assemblable amphiphile.

Aussi, il sera avantageux d'utiliser des polycations, par exemple des polyamines, des histones (protéines entourant l'ADN des cellules eucaryotes et riches en acides aminés basiques), du chitosan, de la polylysine... lorsque le composé auto assemblable comprend une partie polaire anionique et de choisir des polyanions, comme l'acide hyaluronique, lorsque le composé auto assemblable comprend une partie polaire cationique.

Avantageusement, le polymère hydrophile interagit avec le composé amphiphile auto assemblable au moyen de liaisons hydrogènes.

Pour augmenter la solidité du recouvrement de la surface, et avantageusement pour contrôler la biodégradation, il est possible d'effectuer une réticulation dudit polymère hydrophile, par des réactifs appropriés. L'homme du métier, en fonction du polymère considéré est capable de déterminer les méthodes de réticulation appropriées.

Dans un mode de réalisation avantageux, l'invention concerne un procédé selon la définition précédente, dans lequel le composé amphiphile est de formule I suivante : 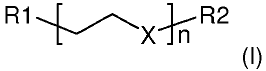 où
- n est différent de zéro,
- X est un groupement fonctionnel choisi parmi, un groupe amino -NR3-, un groupe amido, un groupe imino, un groupe carbonyle, un groupe carboxyle, un groupe ester, un groupe aromatique ou polyaromatique linéaire, un groupe sulfonate, un groupe sulfate, un groupe phosphate ou un groupe phosphonate,
   où R3 est un hydrogène, une amine ou une polyéthylèneimine,
- R2 est un atome d'hydrogène ou un groupement méthyle, éthyle, propyle ou égal à R1,
- et R1 est tel que
   ∘ si n=1, R1 est un alkyle, saturé ou non, en C10-C24, notamment C12-C20, préférentiellement en C14-C18, en particulier C16, et
   ∘ si n>1, R1 est un atome d'hydrogène ou un alkyle, saturé ou non, en C10-C24, notamment C12-C20, préférentiellement en C14-C18, en particulier C16, ou n variant préférentiellement de 8 à 650.

L'auto assemblage du composé amphiphile est assuré par la partie aliphatique ou aromatique de celui-ci. Aussi, plus le composé comprend une partie hydrophobe, plus il est susceptible de s'auto assembler, dans une certaine mesure.

Lorsque n=1 et R2 est un hydrogène, R1 est un alkyle saturé ou non en C10-C24, ce qui signifie que R1 est un alkyle en C6, C7, C8, C9, C10, C11, C12, C13, C14, C15, C16, C17, C18 , C19, C20, C21, C22, C23 ou C24, linéaire ou ramifié.

Les composés avantageux sont choisis parmi les suivants :
- quand X=COO-, l'acide tridécanoïque, l'acide tétradécanoïque, l'acide pentadécanoïque, l'acide hexadécanoïque, l'acide heptadécanoïque, l'acide octadécanoïque, l'acide nonadécanoïque, l'acide eicosanoïque, l'acide hénéicosanoïque, l'acide docosanoïque, l'acide tricosanoïque, l'acide tétracosanoïque, l'acide pentacosanoïque, l'acide hexacosanoïque,
- quand X=NH-, la tridécanamine, la tétradécanamine, la pentadécanamine, l'hexadécanamine, l'heptadécanamine, l'octadecanamine, la nonadécanamine, l'eicosanamine, l'hénéicosanamine, la docosanamine, la tricosanamine, la tétracosanamine, la pentacosanamine et la hexacosanamine, en particulier l'octadécanamine ou stérarylamine est un composé auto assemblable avantageux.

Avantageusement, l'invention concerne un procédé de recouvrement d'une surface hydrophobe par une composition hydrophile, ledit procédé comprenant :
a. une étape de mise en contact de la surface hydrophobe en vue de son recouvrement avec une première composition comprenant
   - un solvant, et
   - un soluté consistant en un composé amphiphile de formule I suivante : 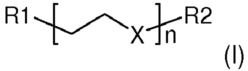 où
      - n étant égal à 1,
      - X est un groupement fonctionnel choisi parmi, un groupe amino, un groupe amido, un groupe carbonyle, un groupe carboxyle, un groupe ester, un groupe aromatique ou polyaromatique linéaire, un groupe sulfonate, un groupe sulfate, un groupe phosphate ou un groupe phosphonate,
      - R2 est un atome d'hydrogène ou un groupement méthyle, éthyle, propyle ou égal à R1,
      - et R1 est un alkyle, saturé ou non, en C10-C24, notamment C12-C20, préférentiellement en C14-C18, en particulier C16,
      ledit solvant étant compatible avec ledit composé et ladite surface hydrophobe,
b. une étape subséquente de rinçage avec une solution aqueuse de ladite surface hydrophobe recouverte à l'étape précédente, et
c. une étape de mise en contact de la surface rincée avec une seconde composition hydrophile comprenant un polymère hydrophile.

Dans un autre mode de réalisation avantageux, l'invention concerne le procédé susmentionné, dans lequel le composé amphiphile choisi parmi les composés de formule suivante : 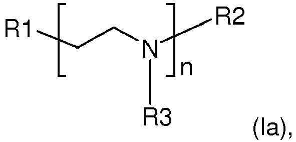 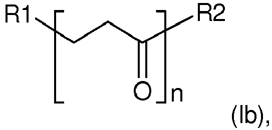 ou 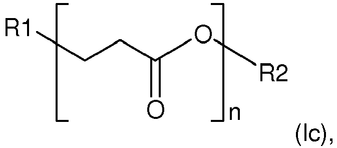 dans lesquelles R1 et R2 sont telles que définies précédemment, et où R3 est un atome d'hydrogène, une amine, une imine, un acide, un alcool, un polymère notamment la polyéthylèneimine.

Dans un autre mode de réalisation, l'invention concerne un procédé selon la définition susmentionnée, dans lequel ledit composé amphiphile est choisi parmi les composés de formules suivantes : 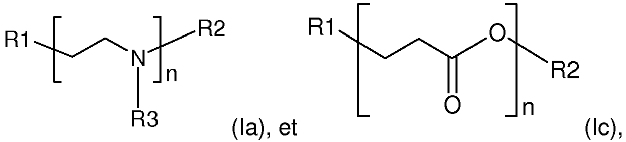 où R2 et R3 sont des atomes d'hydrogène, et R1 est un alkyle, saturé ou non, en C10-C24, notamment C12-C20, préférentiellement en C14-C18, en particulier C16.

Dans encore un autre mode de réalisation avantageux, l'invention concerne le procédé susmentionné, dans lequel le composé amphiphile est de formule 1a, tel que
- si n=1, R2 et R3 sont des atomes d'hydrogène, et R1 est un alkyle en C10-C24, linéaire ou ramifié, en particulier linéaire, et
- si n>1, R1, R2 et R3 sont des atomes d'hydrogène ou des polyéthylèneimine.

Lorsque n>1, et notamment de 8 à 650, des composés avantageux de l'invention sont des polyéthylèneimines linéaires ou branchées, éventuellement des dendrimères. Des exemples de polyéthylèneimine utilisées dans le cadre de l'invention sont présentés ci-après : 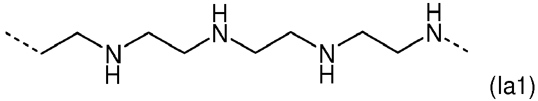 polyéthylèneimine linéaire 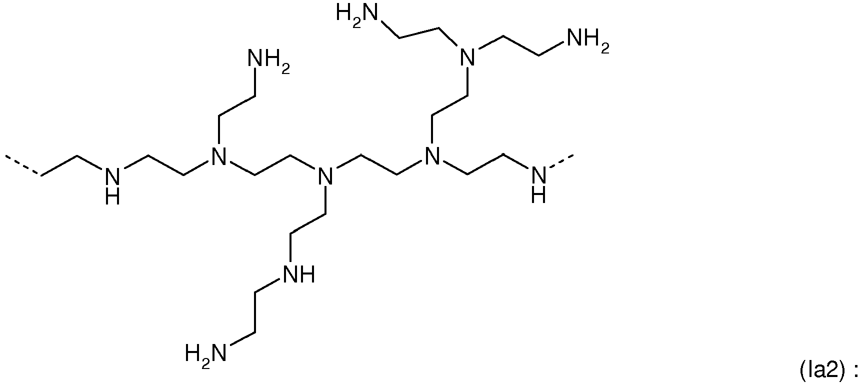 polyéthylèneimine ramifiée, et 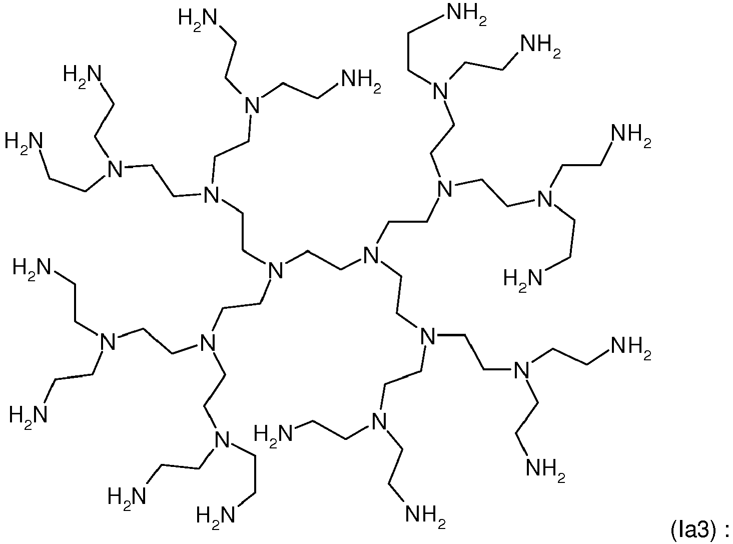 dendrimère d'ordre 4 de polyéthylèneimine.

Les polyéthylèneimines utilisées dans le cadre de l'invention peuvent également être modifiées afin de réduire leur relative toxicité. Notamment de la polyéthylèneimine conjuguée avec du polyéthylène glycol peut être avantageusement utilisé dans le cadre du procédé selon l'invention.

Dans un mode de réalisation avantageux, l'invention concerne le procédé tel que défini ci-dessus, dans lequel ledit composé est de formule la.

Dans un autre mode de réalisation avantageux l'invention concerne le procédé tel que défini ci-dessus, où ledit composé est choisi parmi la stéarylamine ou l'acide stéarique, ou un sel de ceux-ci, ou dans lequel ledit composé est la polyéthylèneimine branchée, avantageusement de bas poids moléculaire, notamment d'environ 10 à environ 100 kDa, préférentiellement d'environ 20 à environ 80 KDa, en particulier d'environ de 25 kDa à 40 kDa, en particulier 25 kDa.

Dans un mode de réalisation avantageux, l'invention concerne un procédé de recouvrement d'une surface hydrophobe par une composition hydrophile, ledit procédé comprenant :
a. une étape de mise en contact de la surface hydrophobe en vue de son recouvrement avec une première composition comprenant
   - un solvant, et
   - un soluté consistant en un composé amphiphile capable de s'auto assembler et d'interagir avec ladite surface, l'auto-assemblage dudit composé et l'interaction avec la surface se faisant au moyen de liaisons différentes de liaisons covalentes ou ioniques, ledit composé étant choisi parmi la stéarylamine ou la polyéthylèneimine, notamment de bas poids moléculaire,
      ledit solvant étant compatible avec ledit composé et ladite surface hydrophobe,
b. une étape subséquente de rinçage avec une solution aqueuse de ladite surface hydrophobe recouverte à l'étape précédente, et
c. une étape de mise en contact de la surface rincée avec une seconde composition hydrophile comprenant un polymère hydrophile.

Un autre mode de réalisation avantageux de l'invention concerne un procédé tel que défini précédemment, où ledit composé amphiphile est choisi parmi la stéarylamine ou l'acide stéarique, ou un sel ou un dérivé de ceux-ci.

La stéarylamine ou l'acide stéarique sont avantageux car ils ne sont pas toxiques pour la santé humaine ou animale. Contrairement à d'autres composés amphiphiles autoassemblables de l'invention, les composés de formule I(a) ou I(c) où R2 et R3 sont des atomes d'hydrogène, et R1 est un alkyle, saturé ou non, en C10-C24, notamment C12-C20, préférentiellement en C14-C18, en particulier C16, et donc la stéarylamine ou l'acide stéarique, peuvent être ingérés ou appliqués sur le corps humain ou animal sans danger.

Il est également avantageux d'utiliser des sels d'acide stéarique qui ne présentent pas non plus de toxicité. Les ions associés aux sels de l'invention sont notamment les ions alcalins tels que les ions lithium (Li⁺), sodium (Na⁺), potassium (K⁺), les ions alcalinoterreux tels que les ions calcium (Ca²⁺), magnésium (Mg²⁺), fer II (Fe²⁺), zinc (Zn²⁺) et les ions trivalents tels que les ions aluminum (Al³⁺) et chrome III (Cr³⁺).

L'acide stéarique peut également être associé à des molécules cationiques telles que des ions ammoniums, notamment des ammoniums quaternaires, des amines primaires ou secondaires protonnées, cette liste n'étant pas limitative.

Avantageusement, l'invention concerne un procédé tel que défini ci-dessus, dans lequel ledit polymère hydrophile est choisi parmi de l'acide hyaluronique, un alginate, la cellulose oxydée, du chitosan ou de l'amidon oxydé ou un de leurs dérivés. Ces polymères sont donnés à titre d'exemple, et cette liste ne devrait pas être considérée comme limitative.

L'acide hyaluronique ou les dérivés de celui-ci sont particulièrement avantageux dans le cadre de dispositifs à usage médical.

Les dérivés de l'acide hyaluronique susmentionnés peuvent être par exemple, sans pour autant limiter la portée de l'invention, l'acide hyaluronique oxydé obtenu par traitement de l' acide hyaluronique au periodate de sodium, tel que cité dans Kristiansen K.A. et al. [Carbohydrate Research 2010, 345,1264-1271], ou encore l'acide hyaluronique modifié par adipique dihydrazide en présence d'un agent de couplage comme décrit dans Prestwich G.D. et al. [Journal of Controlled Release 1998, 53, 93-103].

L'homme du métier peut également se référer aux documents suivants pour déterminer les modifications appropriées de l'acide hyaluronique : Bullpitt P et Aeschlimann D, J. Biomed Mater Res. 1999, 47, 152-169 et Schanté CE et al. Carbohydrate Polymers 2011,85,469-489.

Lorsque le composé amphiphile est l'acide stéarique, et dans le cas du recouvrement par de l'acide hyaluronique, il est avantageux d'utiliser des cations multivalents (par exemple des ions chrome III, qui ne sont pas toxiques, ou des ions aluminium) qui jouent le rôle d'agents de réticulation. Avantageusement, ces cations sont appliqués sur la surface recouverte par le composé amphiphile avant l'enduction avec le polymère hydrophile.

Avantageusement, l'invention concerne le procédé tel que défini ci-dessus, ledit polymère hydrophile est l'acide hyaluronique à une concentration de **0**,1 à 0,5 % ou de 0,1 à 2% dans de l'eau.

Dans un mode de réalisation encore plus avantageux, l'invention concerne un procédé de recouvrement d'une surface hydrophobe par une composition hydrophile selon l'une quelconque des revendications précédentes, ledit procédé comprenant
a. une étape de mise en contact de la surface hydrophobe avec une première composition comprenant, ou essentiellement constituée,
   - d'un solvant approprié, notamment de l'heptane, du dichlorométhane, du 2-méthyl tétrahydrofurane, de la diméthylformamide ou de la diméthylacétamide, ou un mélange de ceux-ci, notamment de la diméthylformamide en mélange avec du dichlorométhane, et
   - de la stéarylamine, notamment à une concentration de 0,1 à 0,5 % ou de 0,1 à 3%, de préférence à 0,3 %, dans ledit solvant,
   ledit solvant étant compatible avec ledit composé et ladite surface hydrophobe,
b. une étape de rinçage à l'eau de ladite surface hydrophobe recouverte à l'étape précédente, et
c. une étape de mise en contact de la surface obtenue à l'étape précédente avec une seconde composition d'acide hyaluronique à une concentration de 0,1 à 0,5 % ou de 0,1 à 2% dans de l'eau.

Dans un mode de réalisation encore plus avantageux, l'invention concerne un procédé de recouvrement d'une surface hydrophobe par une composition hydrophile tel que défini ci-dessus, ledit procédé comprenant
a. une étape de mise en contact de la surface hydrophobe avec une première composition comprenant, ou essentiellement constituée,
   - d'un solvant approprié, notamment de l'heptane, du dichlorométhane, du 2-méthyl tétrahydrofurane, de la diméthylformamide ou de la diméthylacétamide, ou un mélange de ceux-ci, notamment de la diméthylformamide en mélange avec du dichlorométhane, et
   - de l'acide stéarique à une concentration de 0,1 à 3 %, de préférence à 0,3 %, dans ledit solvant,
   ledit solvant étant compatible avec ledit composé et ladite surface hydrophobe,
b. une étape de rinçage à l'eau de ladite surface hydrophobe recouverte à l'étape précédente, et
c. une étape de mise en contact de la surface obtenue à l'étape précédente avec une seconde composition d'acide hyaluronique à une concentration de 0,1 à 0,5 % ou de 0,1 à 2% dans de l'eau.

Dans encore un mode de réalisation encore plus avantageux, l'invention concerne un procédé de recouvrement d'une surface hydrophobe par une composition hydrophile tel que défini ci-dessus, ledit procédé comprenant
a. une étape de mise en contact de la surface hydrophobe avec une première composition comprenant, ou essentiellement constituée,
   - d'un solvant approprié, notamment de l'heptane, du dichlorométhane, du 2-méthyl tétrahydrofurane, de la diméthylformamide ou de la diméthylacétamide, ou un mélange de ceux-ci, notamment de la diméthylformamide en mélange avec du dichlorométhane, et
   - de l'acide stéarique ou de la stéarylamine à une concentration de 0,1 à 3 %, de préférence à 0,3 %, dans ledit solvant,
   ledit solvant étant compatible avec ledit composé et ladite surface hydrophobe,
b. une étape de rinçage à l'eau de ladite surface hydrophobe recouverte à l'étape précédente, et
c. une étape de mise en contact de la surface obtenue à l'étape précédente avec une seconde composition comprenant de l'acide hyaluronique modifié par oxydation, ou de l'acide hyaluronique modifié par de l'adipyl dihydrazide, ou de la cellulose oxydée, ou un mélange de ceux-ci à une concentration de 0,1 à 0,5 % ou de 0,1 à 2% dans de l'eau.

Dans l'invention « de l'acide hyaluronique modifié par oxydation, de l'acide hyaluronique modifié par de l'adipyl dihydrazide, ou de la cellulose oxydée, ou un mélange de ceux-ci » signifie que les compositions suivantes peuvent être envisagées :
- de l'acide hyaluronique modifié par oxydation,
- de l'acide hyaluronique modifié par de l'adipyl dihydrazide,
- de la cellulose oxydée,
- de l'acide hyaluronique modifié par oxydation et de l'acide hyaluronique modifié par de l'adipyl dihydrazide,
- de l'acide hyaluronique modifié par oxydation et de la cellulose oxydée,
- de l'acide hyaluronique modifié par de l'adipyl dihydrazide, et de la cellulose oxydée, et
- de l'acide hyaluronique modifié par oxydation et de l'acide hyaluronique modifié par de l'adipyl dihydrazide et de la cellulose oxydée.

Un autre mode de réalisation, l'invention concerne un procédé de recouvrement d'une surface hydrophobe par une composition hydrophile selon l'une quelconque des revendications précédentes, ledit procédé comprenant
a. une étape de mise en contact de la surface hydrophobe avec une première composition comprenant, ou essentiellement constituée,
   - d'un solvant approprié, notamment de l'eau, du dichlorométhane, du 2-méthyl tetrahydrofurane, de la diméthylformamide, de la diméthylacétamide ou de la n,n'-diméthyle propylène urée (DMPU), ou encore un mélange de ceux-ci, en particulier du dichlorométhane ou du 2-méthyl tetrahydrofurane en mélange avec la diméthylformamide ou de la diméthylacétamide ou de n,n'-diméthyle propylène urée (DMPU), et
   - d'acide stéarique, notamment à une concentration de 0,3 à 1 %, dans ledit solvant,
   ledit solvant étant compatible avec ledit composé et ladite surface hydrophobe,
b. une étape de rinçage à l'eau de ladite surface hydrophobe recouverte à l'étape précédente, et
c. une étape de mise en contact de la surface obtenue à l'étape précédente avec une seconde composition comprenant de l'acide hyaluronique à une concentration de 0,1 à 0,5 % dans de l'eau et des ions chrome III, notamment de l'acétate chrome III basique, en particulier à 0,5% dans l'eau.

Dans un autre mode de réalisation encore plus avantageux, l'invention concerne un procédé de recouvrement d'une surface hydrophobe par une composition hydrophile selon l'une quelconque des revendications précédentes, ledit procédé comprenant d.
une étape de mise en contact de la surface hydrophobe avec une première composition comprenant, ou essentiellement constituée,
   - d'un solvant approprié, notamment de l'eau, du dichlorométhane, du 2-méthyl tetrahydrofurane, de la diméthylformamide, de la diméthylacétamide ou de la n,n'-diméthyle propylène urée (DMPU), ou encore un mélange de ceux-ci, en particulier du dichlorométhane ou du 2-méthyl tetrahydrofurane en mélange avec la diméthylformamide ou de la diméthylacétamide ou de n,n'-diméthyle propylène urée (DMPU)
   - la polyéthylèneimine, notamment à une concentration de 0,3 à 1 %, de préférence à 0,5 %, dans ledit solvant,
   ledit solvant étant compatible avec ledit composé et ladite surface hydrophobe,
e. une étape de rinçage à l'eau de ladite surface hydrophobe recouverte à l'étape précédente, et
f. une étape de mise en contact de la surface obtenue à l'étape précédente avec une seconde composition d'acide hyaluronique à une concentration de 0,1 à 0,5 % dans de l'eau.

Dans le mode de réalisation avantageux ci-dessus, l'étape a. peut être réalisée par incorporation de la stéarylamine à une concentration de 0,1 à 3 %, ou de l'acide stéarique à une concentration de 0,1 à 3 %, dans un matériau en polypropylène avant le moulage. Au refroidissement, la stéarylamine ou l'acide stéarique migrent lentement vers la surface puis s'associe pour former la première couche.

L'invention concerne également une surface hydrophobe recouverte d'acide hyaluronique, susceptible d'être obtenue selon le procédé tel que défini ci-dessus.

La surface recouverte telle que défini ci-dessus, comprend une ou plusieurs couches de composé amphiphile tel que défini ci-dessus, d'une épaisseur déterminée, elle(s)-même recouverte(s) d'une ou plusieurs couche(s) de polymère(s) hydrophile(s).

L'invention concerne également une surface hydrophobe recouverte d'une première couche d'un composé amphiphile capable de s'auto assembler, notamment de la stéarylamine, de l'acide stéarique ou de la polyéthylèneimine, l'auto-assemblage dudit composé et l'interaction avec la surface se faisant au moyen de liaisons différentes de liaisons covalentes ou ioniques, ladite première couche étant recouverte d'une seconde couche comprenant ou essentiellement constituée d'un polymère hydrophile, notamment de l'acide hyaluronique, avantageusement réticulé.

L'invention concerne également l'utilisation d'un complexe de stéarylamine et d'acide hyaluronique pour le recouvrement de surfaces hydrophobes, les composés formant ledit complexe étant liés par des liaisons différentes de liaisons covalentes.

L'invention concerne également l'utilisation d'un complexe de polyéthylèneimine et d'acide hyaluronique pour le recouvrement de surfaces hydrophobes, les composés formant ledit complexe étant liés par des liaisons différentes de liaisons covalentes.

Avantageusement, l'invention concerne une surface hydrophobe recouverte d'une première couche d'un composé amphiphile de formule I suivante : 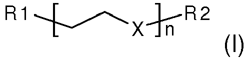 où
- n étant égal à 1,
- X est un groupement fonctionnel choisi parmi, un groupe amino, un groupe amido, un groupe carbonyle, un groupe carboxyle, un groupe ester, un groupe aromatique ou polyaromatique linéaire, un groupe sulfonate, un groupe sulfate, un groupe phosphate ou un groupe phosphonate,
- R2 est un atome d'hydrogène ou un groupement méthyle, éthyle, propyle ou égal à R1,
- et R1 est un alkyle, saturé ou non, en C10-C24, notamment C12-C20, préférentiellement en C14-C18, en particulier C16,
ladite première couche étant recouverte d'une seconde couche comprenant ou essentiellement constituée d'un polymère hydrophile.

Dans l'invention, ledit polymère hydrophile est tel que défini précédemment, et ci-après et inclut la possiblement la présence d'ions, notamment des ions chrome III.

Dans un a autre mode de réalisation avantageux, l'invention concerne une surface hydrophobe telle que définie précédemment, où ledit composé amphiphile est choisi parmi les composés de formules suivantes : 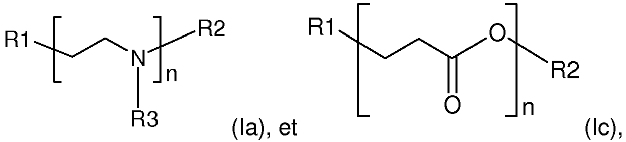 où R2 et R3 sont des atomes d'hydrogène, et R1 est un alkyle, saturé ou non, en C10-C24, notamment C12-C20, préférentiellement en C14-C18, en particulier C16.

Dans encore un autre mode de réalisation, l'invention concerne une surface hydrophobe telle que définie précédemment, où ledit composé amphiphile est choisi parmi la stéarylamine ou l'acide stéarique, ou un sel ou un dérivé de ceux-ci.

Encore plus avantageusement, l'acide stéarique est utilisé à une concentration de 0,1 à 3 %, de préférence à 0,3 %, tandis que la stéarylamine est utilisée à une concentration de 0,1 à 3 %, de préférence 0,3%.

L'invention concerne par ailleurs l'utilisation d'une composition comprenant essentiellement de la stéarylamine ou de l'acide stéarique pour le recouvrement de surfaces hydrophobes par de l'acide hyaluronique, de l'alginate, de la cellulose oxydée, de l'amidon oxydé ou un de leurs dérivés ou un mélange de ceux-ci.

L'invention concerne de plus l'utilisation d'un jeu de compositions pour le recouvrement de surface hydrophobe, ledit jeu de composition comprenant,
- une première composition essentiellement constituée
   - de stéarylamine, notamment une première composition de stéarylamine à une concentration de 0,1 à 0,5 % ou de 0,1 à 3%, dans un solvant approprié ou
   - de polyéthylèneimine, notamment une première composition de polyéthylèneimine de concentration de 0,3 à 1 %, dans un solvant approprié,
   - de l'acide stéarique, notamment une première composition d'acide stéarique à une concentration de 0,1 à 0,5 % ou de 0,1 à 3%, dans un solvant approprié ou
- une seconde composition comprenant ou essentiellement constituée d'acide hyaluronique, ou un de ses dérivés tel que défini ci-dessus, de concentration de 0,1 à 0,5% ou de 0,1 à 2% dans l'eau.

Ledit solvant approprié est notamment de l'eau, de l'heptane, du dichlorométhane, du 2-méthyl tetrahydrofurane, de la diméthylformamide, de la diméthylacétamide ou de la n,n'-diméthyle propylène urée (DMPU), ou encore un mélange de ceux-ci, en particulier du dichlorométhane ou du 2-méthyl tetrahydrofurane en mélange avec la diméthylformamide ou de la diméthylacétamide ou de n,n'-diméthyle propylène urée (DMPU).

En d'autres termes l'invention concerne l'utilisation d'un jeu de compositions pour le recouvrement de surface hydrophobe, ledit jeu de composition comprenant,
- une première composition essentiellement constituée de polyéthylèneimine, notamment de polyéthylèneimine de concentration de 0,3 à 1 %, dans un solvant approprié,
   ledit solvant approprié étant notamment de l'eau, ou un solvant polaire, du dichlorométhane, du 2-méthyl tetrahydrofurane, de la diméthylformamide, de la diméthylacétamide ou de la n,n'-diméthyle propylène urée (DMPU), ou encore un mélange de ceux-ci, en particulier du dichlorométhane ou du 2-méthyl tetrahydrofurane en mélange avec la diméthylformamide ou de la diméthylacétamide ou de n,n'-diméthyle propylène urée (DMPU), et
- une seconde composition comprenant ou essentiellement constituée d'acide hyaluronique de concentration de 0,1 à 0,5% ou de 0,1 à 2% dans l'eau.

Egalement, l'invention concerne l'utilisation d'un jeu de compositions pour le recouvrement de surface hydrophobe, ledit jeu de composition comprenant,
- une première composition essentiellement constituée de stéarylamine, notamment une première composition de stéarylamine à une concentration de 0,1 à 0,5 %, ou de 0,1 à 3% dans un solvant approprié, ou
- une première composition essentiellement constituée d'acide stéarique, notamment une première composition d'acide stéarique à une concentration de 0,1 à 0,5 %, ou de 0,1 à 3% dans un solvant approprié
ledit solvant approprié étant notamment l'heptane, du dichlorométhane, du 2-méthyl tétrahydrofurane, de la diméthylformamide ou de la diméthylacétamide, ou un mélange de ceux-ci, notamment de la diméthylformamide en mélange avec du dichlorométhane, et
- une seconde composition comprenant ou essentiellement constituée d'acide hyaluronique, ou un de ses dérivés tel que défini ci-dessus, notamment l'acide hyaluronique oxydé ou encore l'acide hyaluronique modifié par adipique dihydrazide, ou de la cellulose oxydée, ou un mélange de ceux-ci, de concentration de 0,1 à 0,5% ou de 0,1 à 2% dans l'eau.

Possiblement, le jeu de composition comprend en outre de des ions chrome III.

L'invention concerne en outre l'utilisation d'un jeu de compositions pour le recouvrement de surface hydrophobe, ledit jeu de composition comprenant
a. une première composition essentiellement constituée de stéarylamine ou d'acide stéarique, et
b. une seconde composition comprenant ou essentiellement constituée d'acide hyaluronique, ou l'un de ses dérivés, notamment l'acide hyaluronique oxydé ou encore l'acide hyaluronique modifié par adipique dihydrazide, ou la cellulose oxydée ou un mélange de ceux-ci, de concentration de 0,1 à 2% dans l'eau.

L'invention concerne par ailleurs un kit de recouvrement de surfaces hydrophobes comprenant
- une première composition essentiellement constituée
   - de stéarylamine, notamment une première composition de stéarylamine à une concentration de 0,1 à 0,5 % ou de 0,1 à 3%, dans un solvant approprié ou
   - de polyéthylèneimine, notamment une première composition de polyéthylèneimine de concentration de 0,3 à 1 %, dans un solvant approprié
      ledit solvant approprié étant notamment de l'eau, de l'heptane, du dichlorométhane, du 2-méthyl tetrahydrofurane, de la diméthylformamide, de la diméthylacétamide ou de la n,n'-diméthyle propylène urée (DMPU), ou encore un mélange de ceux-ci, en particulier du dichlorométhane ou du 2-méthyl tetrahydrofurane en mélange avec la diméthylformamide ou de la diméthylacétamide ou de n,n'-diméthyle propylène urée (DMPU), et
- une seconde composition comprenant ou essentiellement constituée d'acide hyaluronique de concentration de 0,1 à 0,5% ou de 0,1 à 2% dans l'eau.

L'invention concerne en outre un kit contenant un contenant comprenant :
- une première composition essentiellement constituée de stéarylamine ou d'acide stéarique, notamment destinés à être dilués dans un solvant approprié à une concentration de 0,1 à 3%,
- un solvant ou plusieurs solvants choisi parmi de l'eau, de l'heptane, du dichlorométhane, du 2-méthyl tetrahydrofurane, de la diméthylformamide, de la diméthylacétamide, de la n,n'-diméthyle propylène urée (DMPU), 1,3-Dioxolane, Cyclopentyl methyle ether ou un mélange de ceux-ci, et
- une seconde composition comprenant, ou essentiellement constituée, d'acide hyaluronique ou l'un de ses dérivés, notamment l'acide hyaluronique oxydé ou encore l'acide hyaluronique modifié par adipique dihydrazide, ou la cellulose oxydée ou un mélange de ceux-ci, de concentration de 0,1 à 2% dans l'eau.

Le kit susmentionné comprend donc trois éléments permettant le recouvrement de surfaces hydrophobes. Le kit comprend par ailleurs une notice d'utilisation sur un support approprié (papier, support informatique, lien internet) permettant de mettre en oeuvre les étapes du procédé de l'invention, tel que défini précédemment.

L'invention sera mieux comprise à la lecture des exemples suivants et des figures annexées. Ces exemples sont fournis à titre d'exemples et ne présentent aucun caractère limitatif de l'invention.

### Légendes des figures

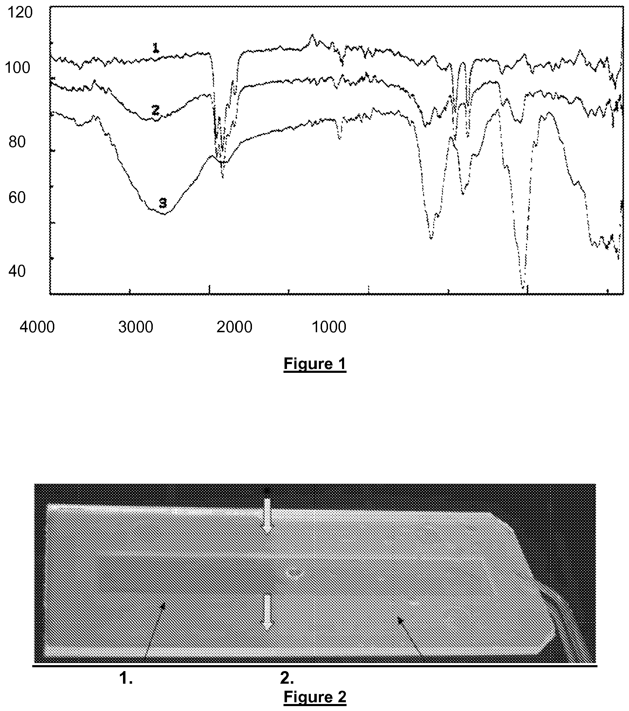

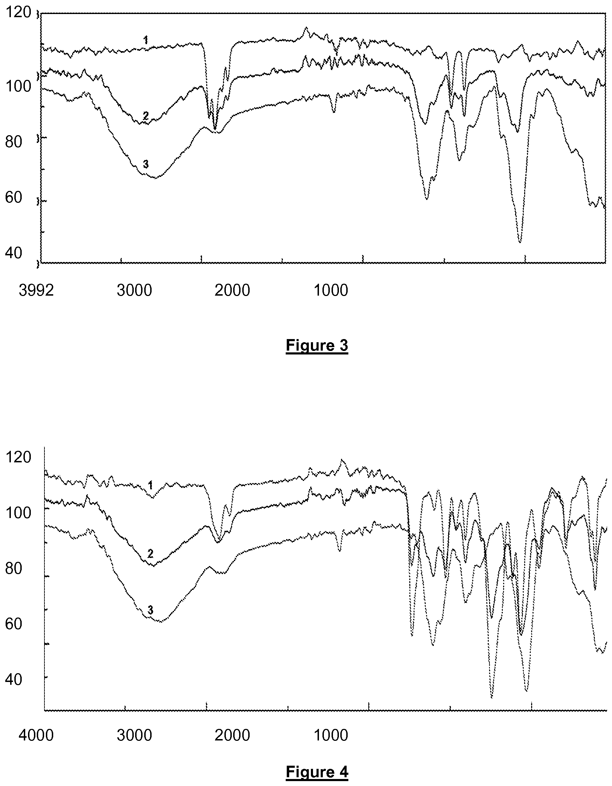

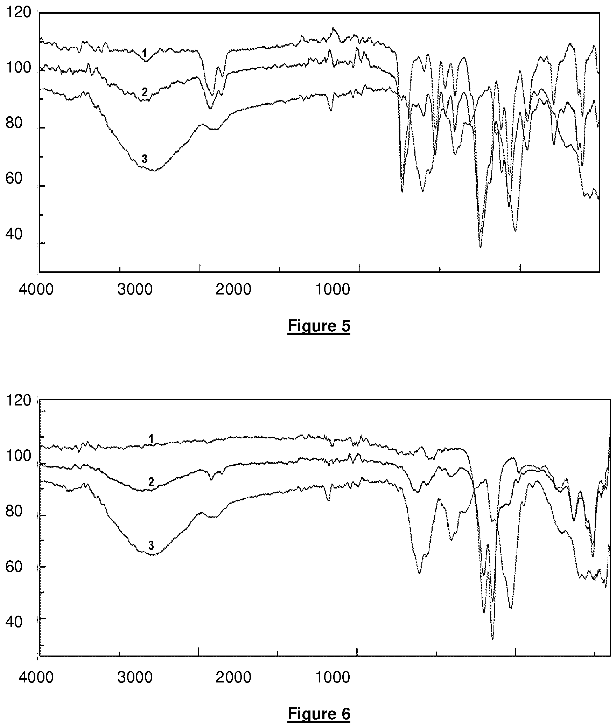

**La** **figure 1** représente un spectre infrarouge par transformée de Fourrier (FT-IR en anglais) d'une surface de polypropylène, tel que décrit dans l'exemple 1, non traitée (courbe 1), traitée selon le procédé de l'invention (courbe 2) et de l'acide hyaluronique seul (courbe 3). L'axe de X représente la longueur d'onde en cm⁻¹ et l'axe des Y représente le coefficient de transmission.
**La** **figure 2** représente une photographie montrant une plaque de polypropylène traitée dans sa partie gauche avec le procédé selon l'invention (comme décrit dans l'exemple 1 ; partie 1. de la photo) et non traitée dans sa partie droite (partie 2. de la photo), et plongée dans l'eau. Les flèches blanches indiquent la zone de séparation entre la partie traitée et la partie non traitée.
**La** **figure 3** représente un spectre infrarouge par transformée de Fourrier d'une surface de polypropylène, telle que décrite dans l'exemple 2, non traitée (courbe 1), traitée selon le procédé de l'invention (courbe 2) et de l'acide hyaluronique seul (courbe 3). L'axe de X représente la longueur d'onde en cm⁻¹ et l'axe des Y représente le coefficient de transmission.
**La** **figure 4** représente un spectre infrarouge par transformée de Fourrier d'une surface de polyuréthane, telle que décrite dans l'exemple 3, non traitée (courbe 1), traitée selon le procédé de l'invention (courbe 2) et de l'acide hyaluronique seul (courbe 3). L'axe de X représente la longueur d'onde en cm⁻¹ et l'axe des Y représente le coefficient de transmission.
**La** **figure 5** représente un spectre infrarouge par transformée de Fourrier d'une surface de polyuréthane, telle que décrite dans l'exemple 4, non traitée (courbe 1), traitée selon le procédé de l'invention (courbe 2) et de l'acide hyaluronique seul (courbe 3). L'axe de X représente la longueur d'onde en cm⁻¹ et l'axe des Y représente le coefficient de transmission.
**La** **figure 6** représente un spectre infrarouge par transformée de Fourrier d'une surface de Téflon®, telle que décrite dans l'exemple 5, non traitée (courbe 1), traitée selon le procédé de l'invention (courbe 2) et de l'acide hyaluronique seul (courbe 3). L'axe de X représente la longueur d'onde en cm⁻¹ et l'axe des Y représente le coefficient de transmission.

### EXEMPLES

### Exemple 1 - Traitement d'une plaquette de polypropylène par la stéarylamine puis enduction par l'acide hyaluronique.

Une plaquette de polypropylène est lavée au préalable par immersion verticalement dans un becher contenant 40ml de dichlorométhane et 40ml de diméthylformamide. Le becher comprenant la plaque a subi un passage aux ultrasons pendant 2 min puis le becher est laissé à agiter sur un agitateur orbital pendant 1 heure à température ambiante (de 17,5°C à 26°C). La plaque est retirée du mélange de lavage et laissée à sécher à l'air libre pendant 15 min. Ensuite, la plaque est trempée verticalement dans un becher contenant 0,3% de stéarylamine dans 20ml de dichlorométhane et 20ml de diméthylformamide. Dans ces conditions, la plaquette est traitée sur la moitié de sa hauteur et elle est placée dans une étuve thermostatée à 26°C sur un agitateur orbital pendant 3 heures. Au bout de ce temps, la plaquette est retirée du becher et elle est trempée dans 3 bains successifs d'eau distillée pour le lavage. Enfin, la plaquette est trempée verticalement dans un becher contenant 80ml d'acide hyaluronique à 0,3% dans l'eau distillée. Le becher est agité sur un agitateur orbital pendant 2 heures à température ambiante. A l'issue de cette période de temps, la plaquette est retirée et laissée à sécher à l'air libre pendant 24 heures.

La surface de la plaquette est analysée par FT-IR **(****figure 1**). Seule la partie préalablement traitée par la stéarylamine (courbe 2) présente des signaux comparables à ceux d'un gel d'acide hyaluronique pris comme référence (courbe 3).

La plaquette séchée est à nouveau trempé dans l'eau pour les besoins d'une photographie (**figure 2**). Deux zones sont clairement distinctes sur cette même plaquette : une zone non traitée par la stéarylamine qui est recouverte de quelques gouttelettes (partie 2.) et une zone correspondant à la partie traitée par la stéarylamine qui est recouverte uniformément d'une couche de gel d'acide hyaluronique (partie 1.).

Ces expériences démontrent que la plaque traitée avec le procédé selon l'invention présente un bon mouillage, alors que la plaque non traitée reste relativement hydrophobe.

Par ailleurs, la présence d'acide hyaluronique recouvrant le substrat est mesurée et dosée par un test colorimétrique selon un protocole adapté de celui décrit par Cesaaretti M. et al. 2003, Carbohydrate Polymers, 54, 59-61.

Le protocole utilisé est le suivant : Une quantité déterminée de l'échantillon est mise en contact et couverte par de l'acide sulfurique 2M (400 microlitres), passée aux ultrasons pendant une minute puis mise à agiter sur agitateur orbital pendant une heure. L'échantillon est retiré de la solution.

A la solution restante est ajouté 400 microlitres de tétraborate de sodium 25 mMolaire dans de l'acide sulfurique concentré. La solution est chauffée à 100 °C pendant 10 minutes puis laissée à refroidir à température ambiante pendant 15 minutes. On ajoute ensuite 200 microlitres d'une solution de carbazole (0,125 % dans l'éthanol absolu). La solution est chauffée une nouvelle fois à 100 °C pendant 10 minutes, puis laissée refroidir à température ambiante

La coloration caractéristique témoigne de la présence d'acide hyaluronique (HA). La quantité d'acide hyaluronique est dosée au spectrophotomètre à une longueur d'onde de 550 nm. Lorsque la surface a été traitée, la solution est de couleur bleue translucide tandis qu'elle est incolore si la surface n'a pas reçu de traitement.

Les résultats obtenus sont représentés dans le tableau 1 suivant :

**Tableau 1**

| **Echantillon** | **DO** |
|---|---|
| Solution sans acide hyaluronique (HA) | 0,2359 |
| Solution contenant 150µg de HA | 0,5824 |
| Polypropylène non traité | 0,2257 |
| Polypropylène traité avec la stéarylamine et enduit de HA | **0,3362** |

Cet autre test confirme la présence de HA sur la surface traitée.

### Exemple 2 - Traitement d'une plaquette de polypropylène par la polyéthylèneimine puis enduction par l'acide hyaluronique.

Une plaquette de polypropylène est lavée au préalable par immersion verticalement dans un becher contenant 80ml d'éthanol. Le becher comprenant la plaque a subi un passage aux ultrasons pendant 2 min puis le becher est laissé à agiter sur un agitateur orbital pendant 1 heure à température ambiante. La plaque est retirée du mélange de lavage et laissée à sécher à l'air libre pendant 15 min. Ensuite, la plaque est trempée verticalement dans un becher contenant 0,5% de polyéthylèneimine dans 40ml d'eau distillé à température ambiante. Dans ces conditions, la plaquette est traitée sur la moitié de sa hauteur et elle est agitée sur un agitateur orbital pendant 3 heures. Au bout de ce temps, la plaquette est retirée du becher et elle est trempée dans 3 bains successifs d'eau distillée pour le lavage. Enfin, la plaquette est trempée verticalement dans un becher contenant 80ml d'acide hyaluronique à 0,3% dans l'eau distillée. Le becher est agité sur un agitateur orbital pendant 2 heures à température ambiante. A l'issue de cette période de temps, la plaquette est retirée et laissée à sécher à l'air libre pendant 24 heures.

La surface de la plaquette est analysée par FT-IR (**figure 3**). Seule la partie préalablement traitée par le polyéthylèneimine (courbe 2) présente des signaux comparables à ceux d'un gel d'acide hyaluronique pris comme référence (courbe 3). Ces expériences démontrent que la plaque traitée avec le procédé selon l'invention présente un bon mouillage, alors que la plaque non traitée reste relativement hydrophobe.

### Exemple 3 - Traitement d'un tube de polyuréthane par la stéarylamine puis enduction par l'acide hyaluronique.

Un tube de polyuréthane est lavé au préalable par immersion verticalement dans un becher contenant 80ml d'heptane. Le becher comprenant le tube a subi un passage aux ultrasons pendant 2 min puis le becher est laissé à agiter sur un agitateur orbital pendant 1 heure à température ambiante. Le tube est retiré du solvant de lavage et laissé à sécher à l'air libre pendant 15 min. Ensuite, le tube est trempé verticalement dans un becher contenant 0,3% de stéarylamine dans 40ml d'heptane. Dans ces conditions, le tube est traité sur la moitié de sa hauteur et il est placé dans une étuve thermostatée à 26°C sur un agitateur orbital pendant 3 heures. Au bout de ce temps, le tube est retiré du becher et il est trempé dans 3 bains successifs d'eau distillée pour le lavage. Enfin, le tube est trempé verticalement dans un becher contenant 80ml d'acide hyaluronique à 0,3% dans l'eau distillée. Le becher est agité sur un agitateur orbital pendant 2 heures à température ambiante. A l'issue de cette période de temps, le tube est retiré et laissé à sécher à l'air libre pendant 24 heures.

La surface du tube est analysée par FT-IR (**figure 4**). Seule la partie préalablement traitée par la stéarylamine (courbe 2) présente des signaux comparables à ceux d'un gel d'acide hyaluronique pris comme référence (courbe 3).

Le tube séché est à nouveau trempé dans l'eau pour le test de mouillabilité. Deux zones sont clairement distinctes: une zone non traitée par la stéarylamine qui est recouverte de quelques gouttelettes et une zone correspondant à la partie traitée par la stéarylamine qui est recouverte uniformément d'une couche de gel d'acide hyaluronique.

Ces expériences démontrent que le tube traité avec le procédé selon l'invention présente un bon mouillage, alors que le tube non traité reste relativement hydrophobe.

### Exemple 4 - Traitement d'un tube de polyuréthane par la polyéthylèneimine puis enduction par l'acide hyaluronique.

Un tube de polyuréthane est lavé au préalable par immersion verticalement dans un becher contenant 80ml d'éthanol. Le becher comprenant le tube a subi un passage aux ultrasons pendant 2 min puis le becher est laissé à agiter sur un agitateur orbital pendant 1 heure à température ambiante. Le tube est retiré du solvant de lavage et laissé à sécher à l'air libre pendant 15 min. Ensuite, le tube est trempé verticalement dans un becher contenant 0,5% de polyéthyleneimine dans 40ml d'eau à température ambiante. Dans ces conditions, le tube est traité sur la moitié de sa hauteur et il est agité sur un agitateur orbital pendant 3 heures. Au bout de ce temps, le tube est retiré du becher et il est trempé dans 3 bains successifs d'eau distillée pour le lavage. Enfin, le tube est trempé verticalement dans un becher contenant 80ml d'acide hyaluronique à 0,3% dans l'eau distillée. Le becher est agité sur un agitateur orbital pendant 2 heures à température ambiante. A l'issue de cette période de temps, le tube est retiré et laissé à sécher à l'air libre pendant 24 heures.

La surface du tube est analysée par FT-IR (**figure 5**). Seule la partie préalablement traitée par la polyéthyleneimine (courbe 2) présente des signaux comparables à ceux d'un gel d'acide hyaluronique pris comme référence (courbe 3).

Le tube séché est à nouveau trempé dans l'eau pour le test de mouillabilité. Deux zones sont clairement distinctes sur cette même plaquette : une zone non traitée par la polyéthyleneimine qui est recouverte de quelques gouttelettes et une zone correspondant à la partie traitée par la polyéthyleneimine qui est recouverte uniformément d'une couche de gel d'acide hyaluronique.

Ces expériences démontrent que le tube traité avec le procédé selon l'invention présente un bon mouillage, alors que le tube non traité reste relativement hydrophobe.

### Exemple 5 - Traitement d'un tube de Teflon® par la stéarylamine puis enduction par l'acide hyaluronique.

Un tube de Teflon® est lavé au préalable par immersion verticalement dans un becher contenant 40ml de dichlorométhane et 40ml de diméthylformamide. Le becher comprenant le tube a subi un passage aux ultrasons pendant 2 min puis le becher est laissé à agiter sur un agitateur orbital pendant 1 heure à température ambiante. Le tube est retiré du mélange de lavage et laissé à sécher à l'air libre pendant 15 min. Ensuite, le tube est trempé verticalement dans un becher contenant 0,3% de stéarylamine dans 20ml de dichlorométhane et 20ml de diméthylformamide. Dans ces conditions, le tube est traité sur la moitié de sa hauteur et il est placé dans une étuve thermostatée à 26 °C sur un agitateur orbital pendant 3 heures. Au bout de ce temps, le tube est retiré du becher et il est trempé dans 3 bains successifs d'eau distillée pour le lavage. Enfin, le tube est trempé verticalement dans un becher contenant 80ml d'acide hyaluronique à 0,3% dans l'eau distillée. Le becher est agité sur un agitateur orbital pendant 2 heures à température ambiante. A l'issue de cette période de temps, le tube est retiré et laissé à sécher à l'air libre pendant 24 heures.

La surface du tube est analysée par FT-IR (**figure 6**). Seule la partie préalablement traitée par la stéarylamine (courbe 2) présente des signaux comparables à ceux d'un gel d'acide hyaluronique pris comme référence (courbe 3).

Le tube séché est à nouveau trempé dans l'eau pour le test de mouillabilité. Deux zones sont clairement distinctes sur cette même plaquette : une zone non traitée par la stéarylamine qui est recouverte de quelques gouttelettes et une zone correspondant à la partie traitée par la stéarylamine qui est recouverte uniformément d'une couche de gel d'acide hyaluronique. Ces expériences démontrent que le tube traité avec le procédé selon l'invention présente un bon mouillage, alors que le tube non traité reste relativement hydrophobe.

### Exemple 6 - Traitement d'un tube de Teflon® par la polyéthylèneimine puis enduction par l'acide hyaluronique.

Un tube de Teflon® est lavé au préalable par immersion verticalement dans un becher contenant 80ml d'éthanol. Le becher comprenant le tube a subi un passage aux ultrasons pendant 2 min puis le becher est laissé à agiter sur un agitateur orbital pendant 1 heure à température ambiante (de 17,5°C à 26°C). Le tube est retiré du solvant de lavage et laissé à sécher à l'air libre pendant 15 min. Ensuite, le tube est trempé verticalement dans un becher contenant 0,5% de polyéthyleneimine dans 40ml d'eau à température ambiante. Dans ces conditions, le tube est traité sur la moitié de sa hauteur et il est agité sur un agitateur orbital pendant 3 heures. Au bout de ce temps, le tube est retiré du becher et il est trempé dans 3 bains successifs d'eau distillée pour le lavage. Enfin, le tube est trempé verticalement dans un becher contenant 80ml d'acide hyaluronique à 0,3% dans l'eau distillée. Le becher est agité sur un agitateur orbital pendant 2 heures à température ambiante. A l'issue de cette période de temps, le tube est retiré et laissé à sécher à l'air libre pendant 24 heures. Le tube séché est à nouveau trempé dans l'eau pour le test de mouillabilité. Deux zones sont clairement distinctes sur cette même plaquette : une zone non traitée par la polyéthyleneimine qui est recouverte de quelques gouttelettes et une zone correspondant à la partie traitée par la polyéthyleneimine qui est recouverte uniformément d'une couche de gel d'acide hyaluronique.

Le test colorimétrique décrit dans l'exemple 1, conduit à l'obtention des résultats figurés dans le tableau 2 suivant :

**Tableau 2**

| **Echantillon** | **DO** |
|---|---|
| Solution sans HA | 0,2359 |
| Solution contenant 150µg de HA | 0,5824 |
| Teflon® non traité | 0,2273 |
| Teflon® traité à la polyéthylèneimine et enduite de HA | 0,3473 |

Ce test montre que le tube de Teflon® recouvert par la polyéthyleneimine est recouvert d'une couche de gel d'acide hyaluronique, alors que la surface non traitée ne l'est pas.

Ces expériences démontrent que le tube traité avec le procédé selon l'invention présente un bon mouillage, alors que le tube non traité reste relativement hydrophobe.

### Exemple 7 - Traitement d'un tube de silicone par la stéarylamine puis enduction par l'acide hyaluronique.

Un tube de silicone est lavé au préalable par immersion verticalement dans un becher contenant 40ml de dichlorométhane et 40ml de diméthylformamide. Le becher comprenant le tube a subi un passage aux ultrasons pendant 2 min puis le becher est laissé à agiter sur un agitateur orbital pendant 1 heure à température ambiante (de 17,5°C à 26°C). Le tube est retiré du mélange de lavage et laissé à sécher à l'air libre pendant 15 min. Ensuite, le tube est trempé verticalement dans un becher contenant 0,3% de stéarylamine dans 20ml de dichlorométhane et 20ml de diméthylformamide. Dans ces conditions, le tube est traité sur la moitié de sa hauteur et il est placé dans une étuve thermostatée à 26°C sur un agitateur orbital pendant 3 heures. Au bout de ce temps, le tube est retiré du becher et il est trempé dans 3 bains successifs d'eau distillée pour le lavage. Enfin, le tube est trempé verticalement dans un becher contenant 80ml d'acide hyaluronique à 0,3% dans l'eau distillée. Le becher est agité sur un agitateur orbital pendant 2 heures à température ambiante. A l'issue de cette période de temps, le tube est retiré et laissé à sécher à l'air libre pendant 24 heures.

Le tube séché est à nouveau trempé dans l'eau pour le test de mouillabilité. Deux zones sont clairement distinctes sur cette même plaquette : une zone non traitée par la stéarylamine qui est recouverte de quelques gouttelettes et une zone correspondant à la partie traitée par la stéarylamine qui est recouverte uniformément d'une couche de gel d'acide hyaluronique.

Le test colorimétrique décrit dans l'exemple 1 donne les résultats indiqués dans le tableau 3 suivant :

**Tableau 3**

| **Echantillon** | **DO** |
|---|---|
| Solution sans HA | 0,2359 |
| Solution contenant 150µg de HA | 0,5824 |
| Silicone non traité | 0,2656 |
| Silicone traité avec de la stéarylamine et enduit avec dl'HA | 0,5416 |

Ce test montre que le tube de silicone recouvert par la stéarylamine est recouvert d'une couche de gel d'acide hyaluronique, alors que la surface non traitée ne l'est pas.

Ces expériences démontrent que le tube traité avec le procédé selon l'invention présente un bon mouillage, alors que le tube non traité reste relativement hydrophobe.

### Exemple 8 - Traitement d'un tube de silicone par la polyéthylèneimine puis enduction par l'acide hyaluronique.

Un tube de silicone est lavé au préalable par immersion verticalement dans un becher contenant 80ml d'éthanol. Le becher comprenant le tube a subi un passage aux ultrasons pendant 2 min puis le becher est laissé à agiter sur un agitateur orbital pendant 1 heure à température ambiante (de 17,5°C à 26°C). Le tube est retiré du solvant de lavage et laissé à sécher à l'air libre pendant 15 min. Ensuite, le tube est trempé verticalement dans un becher contenant 0,5% de polyéthyleneimine dans 40ml d'eau à température ambiante. Dans ces conditions, le tube est traité sur la moitié de sa hauteur et il est agité sur un agitateur orbital pendant 3 heures. Au bout de ce temps, le tube est retiré du becher et il est trempé dans 3 bains successifs d'eau distillée pour le lavage. Enfin, le tube est trempé verticalement dans un becher contenant 80ml d'acide hyaluronique à 0,3% dans l'eau distillée. Le becher est agité sur un agitateur orbital pendant 2 heures à température ambiante. A l'issue de cette période de temps, le tube est retiré et laissée à sécher à l'air libre pendant 24 heures.

Le tube séché est à nouveau trempé dans l'eau pour le test de mouillabilité. Deux zones sont clairement distinctes sur cette même plaquette : une zone non traitée par la polyéthyleneimine qui est recouverte de quelques gouttelettes et une zone correspondant à la partie traitée par la polyéthyleneimine qui est recouverte uniformément d'une couche de gel d'acide hyaluronique.

Le test colorimétrique décrit dans l'exemple 1 donne les rsultats représentés dans le tableau 4 suivant

**Tableau 4**

| **Echantillon** | **DO*** |
|---|---|
| Solution sans HA | 0,2359 |
| Solution contenant 150µg de HA | 0,5824 |
| Silicone non traité | 0,2656 |
| Silicone traité pat la polyéthylèneimine et enduite de HA | 0,3682 |

Ce test montre que le tube de silicone recouvert par la polyéthylèneimine est recouvert d'une couche de gel d'acide hyaluronique, alors que la surface non traitée ne l'est pas.

Ces expériences démontrent que le tube traité avec le procédé selon l'invention présente un bon mouillage, alors que le tube non traité reste relativement hydrophobe.

### Exemple 9 - Traitement d'une barre d'acier inoxydable par la stéarylamine puis enduction par l'acide hyaluronique.

Une barre d'acier inoxydable est lavée au préalable par immersion verticalement dans un becher contenant 40ml de dichlorométhane et 40ml de diméthylformamide. Le becher comprenant la barre a subi un passage aux ultrasons pendant 2 min puis le becher est laissé à agiter sur un agitateur orbital pendant 1 heure à température ambiante (de 17,5°C à 26°C). La barre est retirée du mélange de lavage et laissée à sécher à l'air libre pendant 15 min. Ensuite, la barre est trempée verticalement dans un becher contenant 0,3% de stéarylamine dans 20ml de dichlorométhane et 20ml de diméthylformamide. Dans ces conditions, la barre est traitée sur la moitié de sa hauteur et elle est placée dans une étuve thermostatée à 26°C sur un agitateur orbital pendant 3 heures. Au bout de ce temps, la barre est retirée du becher et elle est trempée dans 3 bains successifs d'eau distillée pour le lavage. Enfin, la barre est trempée verticalement dans un becher contenant 80ml d'acide hyaluronique à 0,3% dans l'eau distillée. Le becher est agité sur un agitateur orbital pendant 2 heures à température ambiante. A l'issue de cette période de temps, la barre est retirée et laissée à sécher à l'air libre pendant 24 heures.

La barre séchée est à nouveau trempée dans l'eau pour le test de mouillabilité. Deux zones sont clairement distinctes sur cette même plaquette : une zone non traitée par la stéarylamine qui est recouverte de quelques gouttelettes et une zone correspondant à la partie traitée par la stéarylamine qui est recouverte uniformément d'une couche de gel d'acide hyaluronique.

Le test colorimétrique décrit dans l'exemple 1 permet également de montrer que la barre d'acier inoxydable recouverte par la stéarylamine est recouverte d'une couche de gel d'acide hyaluronique, alors que la barre non traitée ne l'est pas.

Les résultats obtenus sont indiqués dans le tableau 5 suivant :

**Tableau 5**

| **Echantillon** | **DO** |
|---|---|
| Solution sans HA | 0,2359 |
| Solution contenant 150µg de HA | 0,5824 |
| Acier Inoxydable non traité | 0,2401 |
| Acier Inoxydable traité à la stéarylamine et enduit de HA | 0,5080 |

Ces expériences démontrent que la barre traitée avec le procédé selon l'invention présente un bon mouillage, alors que la barre non traitée reste relativement hydrophobe.

### Exemple 10 - Traitement d'une barre d'acier inoxydable par la polyéthylèneimine puis enduction par l'acide hyaluronique.

Une barre d'acier inoxydable est lavée au préalable par immersion verticalement dans un becher contenant 80ml d'éthanol. Le becher comprenant la barre a subi un passage aux ultrasons pendant 2 min puis le becher est laissé à agiter sur un agitateur orbital pendant 1 heure à température ambiante (de 17,5°C à 26°C). La barre est retirée du solvant de lavage et laissée à sécher à l'air libre pendant 15 min. Ensuite, la barre est trempée verticalement dans un becher contenant 0,5% de polyéthyleneimine dans 40ml d'eau à température ambiante. Dans ces conditions, la barre est traitée sur la moitié de sa hauteur et elle est agitée sur un agitateur orbital pendant 3 heures. Au bout de ce temps, la barre est retirée du becher et elle est trempée dans 3 bains successifs d'eau distillée pour le lavage. Enfin, la barre est trempée verticalement dans un becher contenant 80ml d'acide hyaluronique à 0,3% dans l'eau distillée. Le becher est agité sur un agitateur orbital pendant 2 heures à température ambiante. A l'issue de cette période de temps, la barre est retirée et laissée à sécher à l'air libre pendant 24 heures.

La barre séchée est à nouveau trempée dans l'eau pour le test de mouillabilité. Deux zones sont clairement distinctes sur cette même barre : une zone non traitée par la polyéthyleneimine qui est recouverte de quelques gouttelettes et une zone correspondant à la partie traitée par la polyéthyleneimine qui est recouverte uniformément d'une couche de gel d'acide hyaluronique.

Le test colorimétrique décrit dans l'exemple 1, permet d'obtenir les résultats représentés dans le tableau 6 suivant :

**Tableau 6**

| **Echantillon** | **DO** |
|---|---|
| Solution sans HA | 0,2359 |
| Solution contenant 150µg de HA | 0,5824 |
| Acier Inoxydable non traité | 0,2401 |
| Acier Inoxydable traité par polyéthylèneimine enduit de HA | 0,5685 |

Ce test montre que la barre d'acier inoxydable recouverte par la polyéthylèneimine est recouverte d'une couche de gel d'acide hyaluronique, alors que la barre non traitée ne l'est pas.

Ces expériences démontrent que le tube traité avec le procédé selon l'invention présente un bon mouillage, alors que le tube non traité reste relativement hydrophobe.

### Exemple 11 - Traitement d'une surface COC (Cyclic Olefin Copolymer) par la polyéthylèneimine puis enduction par l'acide hyaluronique.

Un objet en COC est lavé au préalable par immersion verticalement dans un becher contenant 80ml d'éthanol. Le becher comprenant l'objet a subi un passage aux ultrasons pendant 2 min puis le becher est laissé à agiter sur un agitateur orbital pendant 1 heure à température ambiante (de 17,5°C à 26°C). L'objet est retiré du solvant de lavage et laissée à sécher à l'air libre pendant 15 min. Ensuite, l'objet est trempé verticalement dans un becher contenant 0,5% de polyéthyleneimine dans 40ml d'eau à température ambiante. Dans ces conditions, l'objet est traité sur la moitié de sa hauteur et elle est agitée sur un agitateur orbital pendant 3 heures. Au bout de ce temps, l'objet est retiré du becher et il est trempé dans 3 bains successifs d'eau distillée pour le lavage. Enfin, l'objet est trempé verticalement dans un becher contenant 80ml d'acide hyaluronique à 0,3% dans l'eau distillée. Le becher est agité sur un agitateur orbital pendant 2 heures à température ambiante. A l'issue de cette période de temps, l'objet est retiré et laissé à sécher à l'air libre pendant 24 heures.

L'objet séché est à nouveau trempé dans l'eau pour le test de mouillabilité. Deux zones sont clairement distinctes sur cet objet : une zone non traitée par la polyéthyleneimine qui est recouverte de quelques gouttelettes et une zone correspondant à la partie traitée par la polyéthyleneimine qui est recouverte uniformément d'une couche de gel d'acide hyaluronique.

Le test colorimétrique décrit dans l'exemple 1, permet d'obtenir les résultats représentés dans le tableau 7 suivant :

**Tableau 7**

| **Echantillon** | **DO** |
|---|---|
| Solution sans HA | 0,2359 |
| Solution contenant 150µg de HA | 0,5824 |
| COC non traité | 0,2538 |
| COC traité avec la polyéthylèneimine et enduit de HA | 0,3534 |

Ces expériences démontrent que l'objet en COC traité avec le procédé selon l'invention présente un bon mouillage, alors que l'objet non traité reste relativement hydrophobe.

### Exemple 12 - Traitement d'une surface en polytéréphtalate d'éthylène (PET) par la polyéthylèneimine puis enduction par l'acide hyaluronique.

Un objet en PET est lavé au préalable par immersion verticalement dans un becher contenant 80ml d'éthanol. Le becher comprenant l'objet a subi un passage aux ultrasons pendant 2 min puis le becher est laissé à agiter sur un agitateur orbital pendant 1 heure à température ambiante (de 17,5°C à 26°C). L'objet est retiré du solvant de lavage et laissée à sécher à l'air libre pendant 15 min. Ensuite, l'objet est trempé verticalement dans un becher contenant 0,5% de polyéthyleneimine dans 40ml d'eau à température ambiante. Dans ces conditions, l'objet est traité sur la moitié de sa hauteur et elle est agitée sur un agitateur orbital pendant 3 heures. Au bout de ce temps, l'objet est retiré du becher et il est trempé dans 3 bains successifs d'eau distillée pour le lavage. Enfin, l'objet est trempé verticalement dans un becher contenant 80ml d'acide hyaluronique à 0,3% dans l'eau distillée. Le becher est agité sur un agitateur orbital pendant 2 heures à température ambiante. A l'issue de cette période de temps, l'objet est retiré et laissé à sécher à l'air libre pendant 24 heures.

L'objet séché est à nouveau trempé dans l'eau pour le test de mouillabilité. Deux zones sont clairement distinctes sur cet objet : une zone non traitée par la polyéthyleneimine qui est recouverte de quelques gouttelettes et une zone correspondant à la partie traitée par la polyéthyleneimine qui est recouverte uniformément d'une couche de gel d'acide hyaluronique.

Le test colorimétrique décrit dans l'exemple 1 permet d'obtenir les résultats représentés dans le tableau 8 suivant :

**Tableau 8**

| **Echantillon** | **DO*** |
|---|---|
| Solution sans HA | 0,2359 |
| Solution contenant 150µg de HA | 0,5824 |
| PET non traité | 0,2517 |
| PET traité avec la polyétylèneimine et enduit de HA | 0,5137 |

Ce test montre que la surface en PET recouverte par la polyéthylèneimine est recouverte d'une couche de gel d'acide hyaluronique, alors que la barre non traitée ne l'est pas.

Ces expériences démontrent que l'objet traité avec le procédé selon l'invention présente un bon mouillage, alors que l'objet non traité reste relativement hydrophobe.

### Exemple 13 - Récapitulatif

Le tableau 9 suivant résume les différents tests réalisés dans le cadre de l'invention

| **Ex.** | **Surface** | **Lavage** | **Traitement** | **Rinçage à l'eau** | **Enduction avec HA 0.3%** |
|---|---|---|---|---|---|
| 1 | Polypropylène | DCM/DMF (1/1) | 0.3% ODA dans DCM/DMF (1/1) | oui | oui |
| 2 | Polypropylène | Ethanol | 0.5% PEI dans l'eau | oui | oui |
| 3 | Polyuréthane | Heptane | 0.3% ODA dans Heptane | oui | oui |
| 4 | Polyuréthane | Ethanol | 0.5% PEI dans l'eau | oui | oui |
| 5 | Teflon® | DCM/DMF (1/1) | 0.3% ODA dans DCM/DMF (1/1) | oui | oui |
| 6 | Teflon® | Ethanol | 0.5% PEI dans l'eau | oui | oui |
| 7 | Silicone | DCM/DMF (1/1) | 0.3% ODA dans DCM/DMF (1/1) | oui | oui |
| 8 | Silicone | Ethanol | 0.5% PEI dans l'eau | oui | oui |
| 9 | Acier inoxydable | DCM/DMF (1/1) | 0.3% ODA dans DCM/DMF (1/1) | oui | oui |
| 10 | Acier inoxydable | Ethanol | 0.5% PEI dans l'eau | oui | oui |
| 11 | Cyclic Olefin Copolymer (COC) | Ethanol | 0.5% PEI dans l'eau | oui | oui |
| 12 | polytéréphtalate d'éthylène (PET) | Ethanol | 0.5% PEI dans l'eau | oui | oui |

| | | | | | |
|---|---|---|---|---|---|
| Légende : DCM : dichlorométhane, DMF :diméthylformamide, ODA : Stéarylamine (Octadecylamine), PEI : polyethylèneimine, HA : Acide Hyaluronique. | | | | | |

### Exemple 14 - Traitement d'une plaquette de polypropylène par l'acide stéarique puis enduction par l'acide hyaluronique en présence de chrome (III).

### a) traitement

Une plaquette de polypropylène est lavée au préalable par immersion verticalement dans un becher contenant 80ml de diméthylformamide. Le becher comprenant la plaque a subi un passage aux ultrasons pendant 2 min puis le becher est laissé à agiter sur un agitateur orbital pendant 1 heure à température ambiante (de 17,5°C à 26°C). La plaque est retirée du mélange de lavage et laissée à sécher à l'air libre pendant 15 min. Ensuite, la plaque est trempée verticalement dans un becher contenant 0,3% d'acide stéarique dans 40ml de diméthylformamide. Dans ces conditions, la plaquette est traitée sur la moitié de sa hauteur et elle est placée dans une étuve thermostatée à 26°C sur un agitateur orbital pendant 3 heures. Au bout de ce temps, la plaquette est retirée du becher et elle est trempée dans 3 bains successifs d'eau distillée pour le lavage.

### b) enduction

Par la suite, la plaquette est trempée verticalement dans un becher contenant 80ml d'acide hyaluronique à 0,1% et 165 microlitre de Cr(III) Acetate Hydroxide (Acétate de chrome III basique, n°CAS 3943-51-8) à 0.5% dans l'eau distillée. Le becher est laissé dans une étuve à 60°C pendant 2 heures. Enfin, la plaquette est trempée verticalement dans un becher contenant 80ml d'acide hyaluronique à 0,3% dans l'eau distillée. Le becher est agité sur un agitateur orbital pendant 1 heures à température ambiante. A l'issue de cette période de temps, la plaquette est retirée et laissée à sécher à l'air libre pendant 24 heures.

Deux zones sont clairement distinctes sur cet objet : une zone non traitée par l'acide stéarique qui est recouverte de quelques gouttelettes et une zone correspondant à la partie traitée par l'acide stéarique qui est recouverte uniformément d'une couche de gel d'acide hyaluronique.

### Exemple 15 - Traitement d'une plaquette de polypropylène par l'acide stéarique puis enduction par de l'acide hyaluronique substitué par de l'adipyl dihydrazide (AH-ADH)

### a) Préparation de l'acide hyaluronique substitué par de l'adipyl dihydrazide (AH-ADH)

Une solution de 200mg d'acide hyaluronique (Mw 1700KDa) dans 40mL du tampon MES à 50mM (pH 4.6) est préparée. Ensuite, on ajoute 310mg de 1-Ethyl-3-(3-dimethylaminopropyl)carbodiimide (EDC) puis 2.61g d'adipic acid dihydrazide. On laisse la réaction se poursuivre sous agitation magnétique pendant 2 heures à température ambiante. On neutralise le mélange réactionnel en ajoutant NaOH 1N. La purification du produit est faite par la dialyse. La dialyse (membrane cellulosique exclusion Mw 3500) est commencée avec NaCI 100mM pendant 72 heures puis avec l'ethanol 25% et finie avec de l'eau purifiée. Le produit est obtenu par lyophilisation.

La méthode a été décrite précédemment dans dans :Prestwich et al. Journal of Controlled Release 1998, 53, 93-103.

### b) traitement d'une plaquette de Polypropylène

Le traitement est celui décrit à l'exemple 14 à l'étape a).

### c) enduction

Par la suite, la plaquette est trempée verticalement dans un becher contenant 80ml d'acide hyaluronique couplé à de l'adipyl dihydrazide à 0,5% dans l'eau distillée. Le becher est agité sur un agitateur orbital pendant 1 heures à température ambiante. A l'issue de cette période de temps, la plaquette est retirée et laissée à sécher à l'air libre pendant 24 heures.

Deux zones sont clairement distinctes sur cet objet : une zone non traitée par l'acide stéarique qui est recouverte de quelques gouttelettes et une zone correspondant à la partie traitée par l'acide stéarique qui est recouverte uniformément d'une couche de gel d'acide hyaluronique couplé à de l'adipyl dihydrazide.

### Exemple 16 - Traitement d'une plaquette de polypropylène par l'acide stéarique puis enduction par de l'acide hyaluronique substitué par de l'adipyl dihydrazide (AH-ADH) en présence de cellulose oxydée ou d'acide hyaluronique oxydé

### a) préparation de la cellulose oxydée et de l'acide hyaluronique oxydé

### Cellulose :

1g de cellulose régénérée est repris dans 50mL d'eau desioinisée. On ajoute 1,32g de periodate de sodium. Puis, on ajuste le pH du mélange réactionnel à 3.5 en ajoutant HCI 1N. On laisse la réaction se poursuivre sous agitation magnétique pendant 5 heures à 35°C.

### Acide hyaluronique :

200mg d'acide hyaluronique est repris dans 20mL d'eau desionisée. On ajoute 106mg de periodate de sodium. Puis, on ajuste le pH du mélange réactionnel à 3.5 en ajoutant HCI 1N. On laisse la réaction se poursuivre sous agitation magnétique pendant 15 heures à température ambiante. On ajoute 300µL de glycérol dans le mélange réactionnel. La purification du produit est faite par dialyse dans l'eau pendant 72 heures. Le produit est obtenu par lyophilisation.

La méthode a été décrite précédemment dans Kristiansen K.A. et al.Carbohydrate Research 2010, 345,1264-1271

### b) traitement d'une plaquette de Polypropylène

Le traitement est celui décrit à l'exemple 14 à l'étape a).

### c) enduction

Par la suite, la plaquette est trempée verticalement dans un becher contenant un mélange de 40ml d'HA-ADH à 0,5% et 40ml de cellulose oxydée à 0,5% (ou 40ml d'acide hyaluronique oxydé à 0.5%) dans l'eau distillée. Le becher est agité sur un agitateur orbital pendant 15min à température ambiante. A l'issue de cette période de temps, la plaquette est retirée et laissée à sécher à l'air libre pendant 24 heures.

Deux zones sont clairement distinctes sur cet objet : une zone non traitée par l'acide stéarique qui est recouverte de quelques gouttelettes et une zone correspondant à la partie traitée par l'acide stéarique qui est recouverte uniformément d'une couche d'un mélange de gel d'acide hyaluronique couplé à de l'adipyl dihydrazide et de cellulose oxydée ou d'acide hyaluronique oxydé .

## Revendications

1. Procédé de recouvrement d'une surface hydrophobe par une composition hydrophile, ledit procédé comprenant :
a. une étape de mise en contact de la surface hydrophobe en vue de son recouvrement avec une première composition comprenant
- un solvant, et
- un soluté consistant en un composé amphiphile, ledit composé amphiphile est choisi parmi la stéarylamine ou l'acide stéarique, ou un sel ou un dérivé de ceux-ci,
ledit solvant étant compatible avec ledit composé et ladite surface hydrophobe,
b. une étape subséquente de rinçage avec une solution aqueuse de ladite surface hydrophobe recouverte à l'étape précédente, et
c. une étape de mise en contact de la surface rincée avec une seconde composition hydrophile comprenant un polymère hydrophile, ledit polymère hydrophile étant choisi parmi l'acide hyaluronique, l'alginate ou la cellulose oxydée, l'amidon oxydé ou un de leurs dérivés, le collagène, le collagène modifié par oxydation, les polysaccarides, la polylysine, les polyamines et le chitosan.

2. Procédé selon la revendication 1, ledit polymère hydrophile étant l'acide hyaluronique à une concentration de 0,1 à 2 % dans de l'eau.

3. Procédé de recouvrement d'une surface hydrophobe par une composition hydrophile selon l'une quelconque des revendications précédentes, ledit procédé comprenant
a. une étape de mise en contact de la surface hydrophobe avec une première composition comprenant
- un solvant, et
- un soluté consistant en
• soit de la stéarylamine à une concentration de 0,1 à 3 %, de préférence 0,3%,
• soit de l'acide stéarique à une concentration de 0,1 à 3 %, de préférence à 0,3 %,
ledit solvant étant compatible avec ledit composé et ladite surface hydrophobe,
b. une étape de rinçage à l'eau de ladite surface hydrophobe recouverte à l'étape précédente, et
c. une étape de mise en contact de la surface obtenue à l'étape précédente avec une seconde composition d'acide hyaluronique à une concentration de 0,1 à 2 % dans de l'eau.

4. Surface hydrophobe recouverte d'une première couche d'un composé amphiphile, ledit composé amphiphile est choisi parmi la stéarylamine ou l'acide stéarique, ou un sel ou un dérivé de ceux-ci,
ladite première couche étant recouverte d'une seconde couche comprenant ou essentiellement constituée d'un polymère hydrophile, ledit polymère hydrophile étant choisi parmi l'acide hyaluronique, l'alginate, la cellulose oxydée, l'amidon oxydé ou un de leurs dérivés ou un mélange de ceux-ci, le collagène, le collagène modifié par oxydation, les polysaccarides, la polylysine, les polyamines ou le chitosan.

5. Surface hydrophobe recouverte selon la revendication 4, susceptible d'être obtenue par le procédé selon l'une quelconque des revendications 1 à 3.

6. Utilisation d'une composition comprenant essentiellement de la stéarylamine ou de l'acide stéarique pour le recouvrement de surfaces hydrophobes par de l'acide hyaluronique, l'alginate, la cellulose oxydée, l'amidon oxydé ou un de leurs dérivés ou un mélange de ceux-ci.

7. Utilisation selon la revendication 6, pour le recouvrement de surfaces hydrophobes par de l'acide hyaluronique ou l'un de ses dérivés, notamment l'acide hyaluronique oxydé ou encore l'acide hyaluronique modifié par adipique dihydrazide, ou de la cellulose oxydée ou un mélange de ceux-ci, de concentration de 0,1 à 2% dans l'eau.

8. Kit comprenant un contenant comprenant:
- une première composition essentiellement constituée de stéarylamine ou d'acide stéarique,
- un solvant ou plusieurs solvants choisi parmi de l'eau, de l'heptane, du dichlorométhane, du 2-méthyl tetrahydrofurane, de la diméthylformamide, de la diméthylacétamide, de la n,n'-diméthyle propylène urée (DMPU), 1,3-Dioxolane, Cyclopentyl methyle ether ou un mélange de ceux-ci, et
- une seconde composition comprenant, ou essentiellement constituée, d'acide hyaluronique ou l'un de ses dérivés, notamment l'acide hyaluronique oxydé ou encore l'acide hyaluronique modifié par adipique dihydrazide, ou la cellulose oxydée ou un mélange de ceux-ci, de concentration de 0,1 à 2% dans l'eau.

## Patentansprüche

1. Verfahren zum Beschichten einer hydrophoben Oberfläche mit einer hydrophilen Zusammensetzung, wobei das Verfahren umfasst:
a. einen Schritt des Kontaktierens der hydrophoben Oberfläche zum Beschichten mit einer ersten Zusammensetzung, umfassend
- ein Lösungsmittel und
- einen gelösten Stoff, der aus einer amphiphilen Verbindung besteht, wobei die amphiphile Verbindung aus Stearylamin oder Stearinsäure oder einem Salz oder Derivat davon ausgewählt ist,
wobei das Lösungsmittel mit der Verbindung und der hydrophoben Oberfläche kompatibel ist,
b. einen nachfolgenden Spülschritt der im vorherigen Schritt beschichteten hydrophoben Oberfläche mit einer wässrigen Lösung und
c. einen Schritt des Kontaktierens der gespülten Oberfläche mit einer zweiten hydrophilen Zusammensetzung, die ein hydrophiles Polymer umfasst, wobei das hydrophile Polymer aus Hyaluronsäure, Alginat oder oxidierter Cellulose, oxidierter Stärke oder Derivaten davon, Kollagen, oxidativ modifiziertem Kollagen, Polysacchariden, Polylysin, Polyaminen und Chitosan ausgewählt ist.

2. Verfahren nach Anspruch 1, wobei das hydrophile Polymer Hyaluronsäure in einer Konzentration von 0,1 bis 2 % in Wasser ist.

3. Verfahren zum Beschichten einer hydrophoben Oberfläche mit einer hydrophilen Zusammensetzung nach einem der vorangehenden Ansprüche, wobei das Verfahren umfasst
a. einen Schritt des Kontaktierens der hydrophoben Oberfläche mit einer ersten Zusammensetzung, umfassend
- ein Lösungsmittel und
- einen gelösten Stoff, bestehend aus
• entweder Stearylamin in einer Konzentration von 0,1 bis 3 %, vorzugsweise 0,3 %,
• oder Stearinsäure in einer Konzentration von 0,1 bis 3 %, vorzugsweise 0,3 %,
wobei das Lösungsmittel mit der Verbindung und der hydrophoben Oberfläche kompatibel ist,
b. einen Schritt des Spülens der im vorangehenden Schritt abgedeckten hydrophoben Oberfläche mit Wasser und
c. einen Schritt des Kontaktierens der im vorangehenden Schritt erhaltenen Oberfläche mit einer zweiten Zusammensetzung von Hyaluronsäure in einer Konzentration von 0,1 bis 2 % in Wasser.

4. Hydrophobe Oberfläche, die mit einer ersten Schicht einer amphiphilen Verbindung beschichtet ist, wobei die amphiphile Verbindung aus Stearylamin oder Stearinsäure oder einem Salz oder Derivat davon ausgewählt ist,
wobei die erste Schicht mit einer zweiten Schicht bedeckt ist, die ein hydrophiles Polymer umfasst oder im Wesentlichen aus diesem besteht, wobei das hydrophile Polymer aus Hyaluronsäure, Alginat, oxidierter Cellulose, oxidierter Stärke oder Derivaten oder einem Gemisch davon, Kollagen, oxidativ modifiziertem Kollagen, Polysacchariden, Polylysin, Polyaminen oder Chitosan ausgewählt ist.

5. Hydrophobe Oberfläche, beschichtet nach Anspruch 4, erhältlich durch das Verfahren nach einem der Ansprüche 1 bis 3.

6. Verwendung einer Zusammensetzung, die im Wesentlichen Stearylamin oder Stearinsäure umfasst, zum Beschichten hydrophober Oberflächen mit Hyaluronsäure, Alginat, oxidierter Cellulose, oxidierter Stärke oder Derivaten oder einem Gemisch davon.

7. Verwendung nach Anspruch 6 zum Beschichten hydrophober Oberflächen mit Hyaluronsäure oder Derivaten davon, insbesondere oxidierter Hyaluronsäure oder mit Adipinsäuredihydrazid modifizierter Hyaluronsäure oder oxidierter Cellulose oder einem Gemisch davon, in einer Konzentration von 0,1 bis 2 % in Wasser.

8. Kit mit einem Behälter, umfassend:
- eine erste Zusammensetzung, die im Wesentlichen aus Stearylamin oder Stearinsäure besteht,
- ein oder mehrere Lösungsmittel, ausgewählt aus Wasser, Heptan, Dichlormethan, 2-Methyltetrahydrofuran, Dimethylformamid, Dimethylacetamid, N,N'-Dimethylpropylenharnstoff (DMPU), 1,3-Dioxolan, Cyclopentylmethylether oder einem Gemisch davon und
- eine zweite Zusammensetzung, umfassend oder im Wesentlichen bestehend aus Hyaluronsäure oder Derivaten davon, insbesondere oxidierter Hyaluronsäure oder mit Adipinsäuredihydrazid modifizierter Hyaluronsäure, oder oxidierter Cellulose oder einem Gemisch davon, mit einer Konzentration von 0,1 bis 2 % in Wasser.

## Claims

1. A process for covering a hydrophobic surface with a hydrophilic composition, said process comprising:
a. a step of placing in contact of the hydrophobic surface for the purpose of covering it with a first composition comprising
- a solvent, and
- a solute consisting of an amphiphilic compound said amphiphilic compound being chosen from stearylamine or stearic acid, or a salt or a derivative thereof
said solvent being compatible with said compound and said hydrophobic surface,
b. a subsequent step of rinsing with an aqueous solution of said hydrophobic surface covered in the preceding step, and
c. a step of placing in contact of the rinsed surface with a second hydrophilic composition comprising a hydrophilic polymer, said hydrophilic polymer being chosen from hyaluronic acid, alginates, oxidized cellulose, oxidized starch or a derivative thereof, collagen, oxidation-modified collagen, polysaccharides, polylysine, polyamine and chitosan.

2. The process according to claim 1, said hydrophilic polymer being hyaluronic acid at a concentration of from 0.1% to 2% in water.

3. The process for covering a hydrophobic surface with a hydrophilic composition according anyone of previous claims, said process comprising
a. a step of placing in contact of the hydrophobic surface with a first composition comprising
- a solvent, and
- a solute consisting of
• either stearylamine at a concentration of from 0.1% to 3%, preferably 0.3%,
• or stearic acid at a concentration of from 0.1% to 3%, preferably 0.3%,
said solvent being compatible with said compound and said hydrophobic surface,
b. a step of rinsing with water of said hydrophobic surface covered in the preceding step, and
c. a step of placing in contact of the surface obtained in the preceding step with a second hyaluronic acid composition at a concentration of from 0.1% to 2% in water.

4. A hydrophobic surface covered with a first layer of an amphiphilic compound, said amphiphilic compound being chosen from stearylamine or stearic acid, or a salt or a derivative thereof,
said first layer being covered with a second layer comprising or consisting essentially of a hydrophilic polymer, said hydrophilic polymer being chosen from hyaluronic acid, alginates, oxidized cellulose, oxidized starch or a derivative thereof, collagen, oxidation-modified collagen, polysaccharides, polylysine, polyamines or chitosan.

5. The hydrophobic surface according to claim 4, liable to be obtained from the process according to anyone of claims 1 to 3.

6. Use of a composition essentially comprising essentially stearylamine or stearic acid for covering hydrophobic surfaces with hyaluronic acid, alginates, oxidized cellulose, oxidized starch or a derivative thereof, or a mixture thereof.

7. Use according to claim 6, for covering hydrophobic surfaces with hyaluronic acid or a derivative thereof, especially oxidized hyaluronic acid or hyaluronic acid modified with adipic dihydrazide, or oxidized cellulose or a mixture thereof, at a concentration of from 0.1% to 2% in water.

8. A kit comprising a container comprising:
- a first composition consisting essentially of stearylamine or stearic acid,
- a solvent or several solvents chosen from water, heptane, dichloromethane, 2-methyltetrahydrofuran, dimethylformamide, dimethylacetamide, N,n'-dimethylpropylenuerea (DMPU), 1,3-dioxolane or cyclopentyl methyl ether, or a mixture thereof, and
- a second composition comprising, or consisting essentially of, hyaluronic acid or a derivative thereof, especially oxidized hyaluronic acid or hyaluronic acid modified with adipic dihydrazide, or oxidized cellulose or a mixture thereof, at a concentration of from 0.1% to 2% in water.
